# EUROPEAN PATENT APPLICATION

(11) **EP 2 214 006 A2**
(43) Date of publication of application: **04.08.2010**
(21) Application number: 09172173.8
(22) Date of filing: 05.10.2009
(51) Int. Cl.: G01N 27/02, G01N 27/72, G01N 1/28

(54) **Pressure device for measuring physical property**

(30) Priority: 30.01.2009 KR 20090007707
(71) Applicant: SNU R&DB Foundation, Seoul 151-742 (KR)
(72) Inventor: Kim, Kee-Hoon, 150-050, Seoul (KR); Nallaiyan, Manivannan, 151-747, Seoul (KR); Nam, Dong-Hak, 135-927, Seoul (KR); Chun, Sae-Hwan, 151-761, Seoul (KR)
(74) Representative: Capasso, Olga

(57) **Abstract**

The present invention relates to a pressure device for measuring a physical property. A pressure device for measuring a physical property according to an exemplary embodiment of the present invention includes i) a sealing member adapted to incorporate a sample and a pressure transmission medium that surrounds the sample, ii) an inner cylinder that is provided with a through-hole in which the sealing member is installed, iii) an outer cylinder that surrounds the inner cylinder in close contact with an outer surface of the inner cylinder; and iv) a piston joined to the through-hole of the inner cylinder to pressurize the sealing member. The inner cylinder and the outer cylinder are fabricated by a non-magnetic metal. In the pressure device for measuring a physical property, the volume and a lattice structure of the sample are changed by pressurizing the sample with the piston to thereby induce a change of electrical and magnetic properties of the sample.

## Description

### BACKGROUND OF THE INVENTION

### (a) Field of the Invention

The present invention relates to a pressure device for measuring a physical property, and more particularly, to a pressure device for measuring a physical property that pressurizes a sample with being mounted on a physical property measuring system (PPMS) and investigates a change of the volume and a lattice structure of the pressurized sample and changes of an electrical characteristic and a magnetic characteristic.

### (b) Description of the Related Art

Electrical characteristics and magnetic characteristics of a transition metal and an alloy thereof, and a rare-earth compound have been actively researched in a condensed matter physics field. Theses characteristics are very sensitive to the volume and a lattice structure of a sample and are controllable by an external pressure. When the external pressure is continuously applied to the sample to be measured, electrical and magnetic states may be changed by the change of the volume and a lattice structure of the sample.

The pressure applied to the sample is an important element in measuring the physical property. Further, high-pressure and high-magnetic field conditions at a low temperature are very important elements in research fields of a quantum critical behavior and a physical property of a condensed material. Therefore, a pressure device is mounted on a physical property measuring system and is used for pressurizing the sample at a high pressure under low-temperature and high-magnetic field conditions.

Pressure devices having various structures and experimental technologies have been developed. Among them, a device that has a sample inside of a cylinder and applies a pressure to the sample by pressurizing the inside of the cylinder with a piston has been known. The pressure device must secure high durability under the low-temperature, high-pressure, and high-magnetic field conditions and have a structure in which parts and samples can be easily replaced.

The above information disclosed in this Background section is only for enhancement of understanding of the background of the invention and therefore it may contain information that does not form the prior art that is already known in this country to a person of ordinary skill in the art.

### SUMMARY OF THE INVENTION

The present invention has been made in an effort to provide a pressure device for measuring a physical property having advantages of securing high durability under low-temperature, high-pressure, and high-magnetic field conditions by improving mechanical rigidities of a cylinder and a piston, facilitating replacement of parts and samples depending on the experimental conditions for measurement, and effectively bearing up against a repetitive thermal cycling by securing a stable structure with respect to a change in heat and pressure.

An exemplary embodiment of the present invention provides a pressure device for measuring a physical property that includes i) a sealing member adapted to incorporate a sample and a pressure transmission medium that surrounds the sample, ii) an inner cylinder that is provided with a through-hole in which the sealing member is installed, iii) an outer cylinder that surrounds the inner cylinder in close contact with an outer surface of the inner cylinder; and iv) a piston joined to the through-hole of the inner cylinder to pressurize the sealing member. The inner cylinder and the outer cylinder are fabricated by a non-magnetic metal. In the pressure device for measuring a physical property, the volume and a lattice structure of the sample are changed by pressurizing the sample with the piston to thereby induce a change of electrical and magnetic properties of the sample.

At least one of the outer cylinder and the inner cylinder may be fabricated by a nickel-chrome-aluminum alloy. Meanwhile, the outer cylinder may be fabricated by a nickel-chrome-aluminum alloy and the inner cylinder may be fabricated by any one of a cooper-beryllium alloy, maraging steel, and a nickel-cobalt-chrome-molybdenum alloy.

The sealing member may include i) a sealing cap that is in closed contact with the piston and of which an end opposite to the piston is opened; and ii) a support joined to the end of the sealing cap. The sealing cap may be fabricated by polytetrafluoroethylene (PTFE) and the support may be fabricated by the copper-beryllium alloy. An outer diameter of the sealing cap is larger than a diameter of the through-hole, such that the sealing cap may press on the through-hole. A lubricant containing polytetrafluoroethylene (PTFE) powder may be applied between the sealing cap and the inner cylinder.

The inner cylinder may be detachably joined to the outer cylinder. A thread is formed on an outer surface of the inner cylinder and an inner surface of the outer cylinder, such that the inner cylinder and the outer cylinder may be joined to each other by screw engagement.

The outer cylinder may have a first flange formed at a pressure applied end thereof and a second flange formed at the other end opposite to the first flange. The pressure device for measuring a physical property may further include i) a first locking nut that screw-engages with the inside of the first flange to fix a position of the piston and ii) a second locking nut that screw-engages with the inside of the second flange to fix a position of the sealing member.

The piston may include i) a first piston joined to the inner cylinder, ii) a pressure transmission pad connected to the first piston in a pressure applied direction, and iii) a second piston connected to the pressure transmission pad in the pressure applied direction. The pressure transmission pad may have a width larger than the first and second pistons. At least one of the first piston, the pressure transmission pad, and the second piston may be fabricated by tungsten carbide.

The pressure device for measuring a physical property may further include a piston holder that surrounds a pressure applied end of the second piston. The piston holder may include i) a back-up member fixed to the end of the second piston and ii) a holder body that surrounds the entirety of the back-up member and a portion of the second piston.

The back-up member may have a width larger than the second piston. At least one of the second piston and the back-up member may be fabricated by tungsten carbide. The holder body may have a width larger than the back-up member. At least one of the second piston and the back-up member may be fabricated by tungsten carbide and the holder body may be fabricated by stainless steel.

The first locking nut may have a first opening having a shape corresponding to a shape of the pressure transmission pad and a second opening having a shape corresponding to a shape of the second piston. The pressure device for measuring a physical property may further include a fixing member that is fixed to the sealing member and is in closed contact with an end of the inner cylinder in the inside of the second flange. The second locking nut may have an opening having a shape corresponding to a shape of the fixing member. The outer cylinder, the first locking nut, and the second locking nut may be fabricated by the nickel-chrome-aluminum alloy.

The pressure device for measuring a physical property may further include i) a lead wire that passes through the sealing member, the fixing member, and the second locking nut, and is electrically connected to the sample; and ii) a protection tube that surrounds the lead wire with an insulating adhesive interposed therebetween.

The lead wire may be a copper wire and is may be mounted on the protection tube with being twisted. The insulating adhesive may be an epoxy-based adhesive. The protection tube may be fabricated by any one of a polymer resin, cupro-nickel, and stainless steel.

As the pressure device for measuring a physical property has a dual structure of the inner cylinder and the outer cylinder, it is possible to improve durability by securing high mechanical rigidity. In addition, since the inner cylinder is detachably joined to the outer cylinder, it is possible to easily replace the inner cylinder with an inner cylinder having a different material by separating the inner cylinder from the outer cylinder in accordance with an experimental condition.

Further, since the inner cylinder, the outer cylinder, and the locking nuts are fabricate by a high-rigidity and non-magnetic metal, the pressure device can effectively endure a repetitive thermal cycling and perform accurate measurement under a cryogenic high-pressure and high-magnetic field environment.

### BRIEF DESCRIPTION OF THE DRAWINGS

FIG. 1 is a perspective view of a pressure device for measuring a physical property according to an exemplary embodiment of the present invention.
FIG. 2 is a cross-sectional view of a pressure device for measuring a physical property according to an exemplary embodiment of the present invention.
FIG. 3 is a cross-sectional view illustrating a state in which an inner cylinder, a sealing member, and a piston are disassembled in the pressure device shown in FIG. 2.
FIG. 4 is a partial cross-sectional view illustrating another embodiment of a second piston in the pressure device shown in FIG. 2.
FIG. 5 is a partial cross-sectional view illustrating a state in which a first flange and a first locking nut are separated from each other in the pressure device shown in FIG. 2.
FIG. 6 is a partial cross-sectional view illustrating a state in which a second flange and a second locking nut are separated from each other in the pressure device shown in FIG. 2.
FIG. 7 is a partial cross-sectional view illustrating a sealing member, a lead wire, and a protection tube in the pressure device shown in FIG. 2.
FIG. 8 is a partial cross-sectional view illustrating another embodiment of an inner cylinder and an outer cylinder in the pressure device shown in FIG. 2.

### DETAILED DESCRIPTION OF THE EMBODIMENTS

When an element such as a layer, film, region, or substrate is referred to as being "on" another element, it can be directly on the other element or intervening elements may also be present. On the contrary, when an element such as a layer, film, region, or substrate is referred to as being "directly on", intervening elements are not present.

Terms representing relative spaces such as "below", "on", etc. may be used for more easily describing a relationship between any one part and the other part that are shown in the figure. These terms are intended to include other meanings or operations of a used device altogether with meanings intended in the figure. For example, when the device of the figure is turned over, any elements described to be positioned "below" another element is described to be positioned "on" other elements. Accordingly, an exemplary term such as "below" includes all downward elements. The device can rotate at 90 degrees or another angle and a term representing a relative space is also analyzed thereby.

Special terms used herein are just used for referring a specific embodiment, but are not intended to limit the present invention. Singular types used herein include plural types as long as words do not represent a meaning clearly contrary thereto. A meaning "include" used in the specification embodies a specific characteristic, region, , step, operation, element, and/or component and does not except existence or addition of another specific characteristic, region, step, operation, element, and/or group.

Although differently not defined, all terms including technical terms and scientific terms used herein have the same meanings as meanings understood by those skilled in the art. Terms defined in dictionaries, which are used in general dictionaries are analyzed to have meanings that coincide with relevant technical documents and currently disclosed contents. As long as the terms not defined, the terms not analyzed as ideal meanings or official meanings.

The exemplary embodiments of the present invention, which are described by referring to cross-sectional views shows preferred embodiments of the present invention in detail. As a result, various modifications of diagrams, for example, modifications of manufacturing methods and/or specifications are expected. Accordingly, the exemplary embodiments are not limited to a specific form of an illustrated region and include modifications of forms by manufacturing. Therefore, the regions shown in the figure are originally approximate, forms thereof are intended to illustrate accurate forms of the regions, but limit the scope of the present invention.

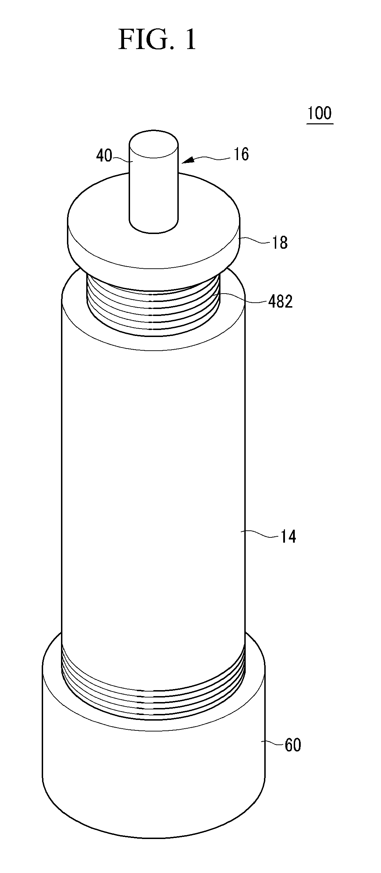

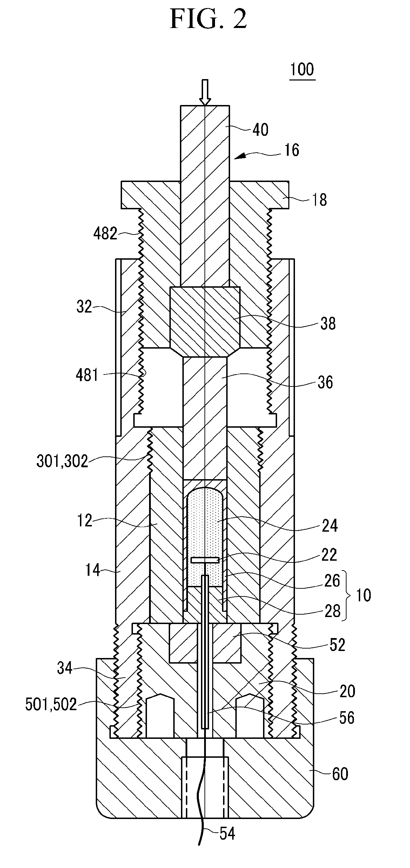

FIG. 1 is a perspective view of a pressure device 100 for measuring a physical property according to an exemplary embodiment of the present invention. FIG. 2 is a cross-sectional view of a pressure device 100 for measuring a physical property according to an exemplary embodiment of the present invention. The structure of the pressure device 100 shown in FIGS. 1 and 2 just exemplifies the present invention, but the present invention is not limited thereto. Therefore, the structure of the pressure device 100 may be modified into other forms.

Referring to FIGS. 1 and 2, the pressure device 100 for measuring a physical property includes a sealing member 10, an inner cylinder 12, an outer cylinder 14, a piston 16, a first locking nut 18, and a second locking nut 20.

The sealing member 10 incorporates a sample 22 which is a measurement target material and a pressure transmission medium 24 surrounding the sample 22. The sealing member 10 includes a sealing cap 26 of which a lower end is opened and a support 28 joined to the lower end of the sealing cap 26. The sealing cap 26 is directly applied with a pressure from the piston 16. This pressure is transmitted to the sample 22 through the pressure transmission medium 24 and induces changes of electrical and magnetic states by changing the volume and a lattice structure of the sample 22.

The sealing cap 26 may be fabricated by a polymer resin and the support 28 may be fabricated by a high-rigidity metal. For example, the sealing cap 26 may be fabricated by polytetrafluoroethylene (PTFE) having excellent thermal and chemical properties and the support 28 may be fabricated by a copper-beryllium (CuBe) alloy having excellent fatigue-resistant, wear-resistant, and corrosion-resistant properties.

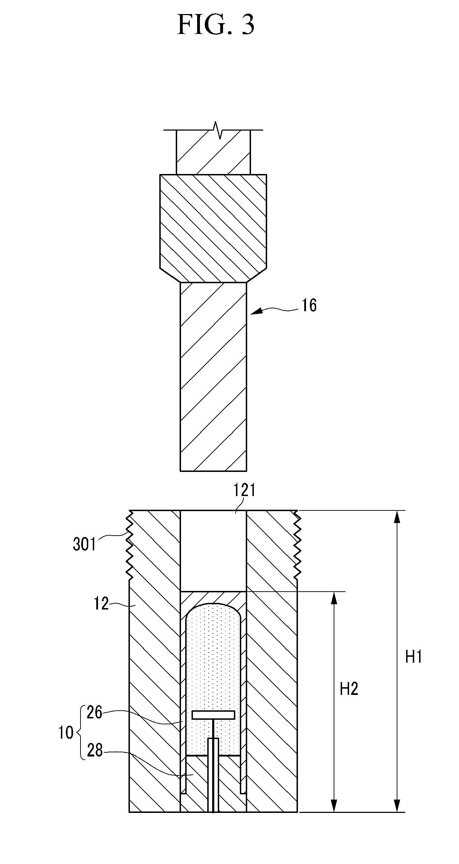

FIG. 3 is a cross-sectional view illustrating state in which the inner cylinder 12, the sealing member 10, and the piston 16 are disassembled in the pressure device 100 shown in FIG. 2.

Referring to FIG. 3, the inner cylinder 12 is provided with a through-hole 121 in which the sealing member 10 is installed. The through-hole 121 is configured to pass through the inner cylinder 12 in a vertical direction of FIG. 3. A height H1 of the inner cylinder 12 is higher than a height H2 of the sealing member 10. The sealing member 10 is positioned below the through-hole 121 of the inner cylinder 12. Therefore, a space into which the piston 16 is inserted is provided above the sealing member 10.

An outer diameter of the sealing cap 26 may be formed a bit larger than a diameter of the through-hole 121. Accordingly, when the sealing cap 26 is pushed into the through-hole 121, the sealing cap 26 presses on the through-hole 121, such that the sealing cap 26 can be firmly fixed to the through-hole 121. At this time, as surface roughness of the sealing cap 26 increases, the sealing cap 26 may be torn by pressurization through the piston 16. Therefore, the sealing cap 26 preferably has small surface roughness as possible.

In the pressurization through the piston 16, friction may be generated between the sealing cap 26 and the inner cylinder 12. In order to reduce the friction, the through-hole 121 of the inner cylinder 12 may be polished or a high-pressure lubricant containing polytetrafluoroethylene (PTFE) powder may be applied between the sealing cap 26 and the inner cylinder 12.

Referring back to FIG. 2, the outer cylinder 14 surrounds the inner cylinder 12 in close contact with an outer surface of the inner cylinder 12. Mechanical rigidity and durability of the pressure device 100 can be enhanced by a dual structure of the inner and outer cylinders 12 and 14. For example, a crack may be generated toward the outer surface of the inner cylinder 12 from the through-hole 121 (see FIG. 3) of the inner cylinder 12 in the pressurization. In this case, the outer cylinder 14 suppresses propagation of the crack of the inner cylinder 12 to thereby enhance the durability of the pressure device 100.

In addition, the inner cylinder 12 and the outer cylinder 14 are detachably joined to each other. Accordingly, the inner cylinder 12 can be easily separated and replaced from the outer cylinder 14 in accordance with experimental conditions.

For example, a first thread 301 may be formed at a portion of the outer surface of the inner cylinder 12 and a second thread 302 may be formed at a portion of an inner surface of the outer cylinder 14 corresponding thereto. The first and second threads 301 and 302 may be positioned on an upper part of the inner and outer cylinders 12 and 14 which are parts to which a pressure is applied. The inner cylinder 12 is firmly fixed to the inside of the outer cylinder 14 by screw engagement and may be easily separated from the outer cylinder 14 if needed.

The outer cylinder 14 is fabricated by a non-magnetic high-rigidity metal.
For example, the outer cylinder 14 may be fabricated by a nickel-chrome-aluminum (NiCrAl) alloy. The nickel-chrome-aluminum alloy has high durability under low-temperature, high-pressure, and high-magnetic field conditions and has high adaptability to a magnetic field environment which rapidly changes.

In particular, since the nickel-chrome-aluminum alloy includes aluminum, the nickel-chrome-aluminum alloy has lower magnetic susceptibility than a compatibilized MP35N alloy (nickel-cobalt-chrome-molybdenum alloy) and shows more excellent performance under the high-magnetic field condition. In addition, the nickel-chrome-aluminum alloy has rigidity higher than maraging steel having high rigidity in spite of high magnetic susceptibility at a low temperature. The nickel-chrome-aluminum alloy used for the outer cylinder 14 has Rockwell rigidity of 52.

Further, a reinforced nickel-chrome-aluminum alloy which is aged at 850°C can increase a maximum pressure which can be achieved by the piston up to 4 GPa at 4.2 K. The nickel-chrome-aluminum alloy has an electrical resistance of approximately 90 µΩcm at 300 K and an electrical resistance of approximately 50 µΩcm at 4.2 K. As a slight change of the electrical resistance caused by a temperature change, an electrical structure of the nickel-chrome-aluminum alloy is slightly changed by applying a high magnetic field. Accordingly, the outer cylinder 14 fabricated by the nickel-chrome-aluminum alloy can maintain high durability even under the high-magnetic field environment.

The inner cylinder 12 may be fabricated by the nickel-chrome-aluminum alloy similarly as the outer cylinder 14. Meanwhile, the inner cylinder 12 may be fabricated by a material different from the material of the outer cylinder 14 depending on experimental conditions such as temperature and pressure ranges, etc. For example, the inner cylinder 12 may be fabricated by any one among the copper-beryllium alloy, the maraging steel, and the nickel-cobalt-chrome-molybdenum alloy.

In the outer cylinder 14, a first flange 32 is formed at an end (upper end of FIG. 2) of a portion to which the pressure is applied and a second flange 34 is formed at an opposite end (lower end of FIG. 2) to the first flange 32. Outer diameters of the first and second flanges 32 and 34 are the same as an outer diameter of the outer cylinder 14. A predetermined space is formed inside of the first and second flanges 32 and 34.

The piston 16 may include a first piston 36, a pressure transmission pad 38, and a second piston 40. The first piston 36 is joined to the through-hole 121 (see FIG. 3) of the inner cylinder 12 to pressurize the sealing cap 26. The pressure transmission pad 38 is fixed to an upper end of the first piston 36 and the second piston 40 is fixed to an upper end of the pressure transmission pad 38. The pressure transmission pad 38 may have a width larger than the first and second pistons 36 and 40.

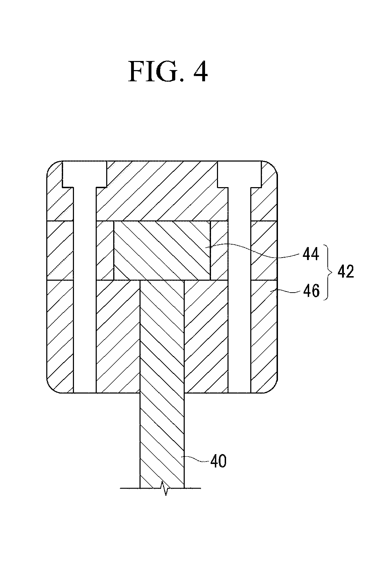

The first and second pistons 36 and 40 and the pressure transmission pad 38 may be fabricated by the non-magnetic high-rigidity metal, i.e., tungsten carbide. The second piston 40 can be directly applied with the pressure from a hydraulic ram (not shown) in close contact with the hydraulic ram. Meanwhile, as shown in FIG. 4, the second piston 40 is joined to a piston holder 42 to receive the pressure from the hydraulic ram through the piston holder 42.

Referring to FIG. 4, the piston holder 42 includes a back-up member 44 connected to an upper end of the second piston 40 and a holder body 46 that surrounds the entirety of the back-up member 44 and a portion of an upper part of the second piston 40. The back-up member 44 has a width larger than the second piston 40 and may be fabricated by the same material as the second piston 40. The holder body 46 has a width larger than the back-up member 44 and may be fabricated by a different material from the materials of the second piston 40 and the back-up member 44. For example, the holder body 46 may be fabricated by stainless steel having high rigidity and corrosion resistance.

When the second piston 40 is directly applied with the pressure from the hydraulic ram, the first piston 36 may be broken while scratching an inner wall of the inner cylinder 12 (see FIG. 2) even though the first piston 36 (see FIG. 2) and the second pistons 40 are deviated from the inner cylinder 12. However, when the piston holder 42 is used, the second piston 40 can be stably applied with the pressure, such that it is possible to prevent the first piston 36 from being broken.

Referring to FIG. 2, the first locking nut 18 is detachably joined to the inside of the first flange 32. For this, a third thread 481 may be formed on an inner wall of the first flange 32 and a fourth thread 482 may be formed on an outer wall of the first locking nut 18. The first locking nut 18 serves to maintain the pressure with the piston 16 by fixing a position of the piston 16 pressurized by the hydraulic ram even after the hydraulic ram is separated from the piston 16.

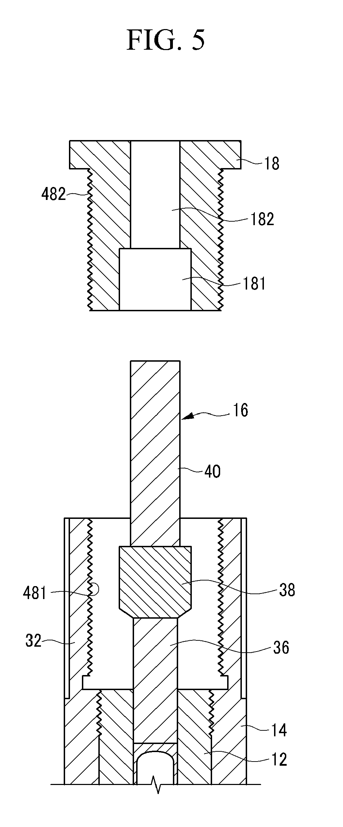

FIG. 5 is a partial cross-sectional view illustrating a state in which the first flange 32 and the first locking nut 18 are separated from each other in the pressure device 100 shown in FIG. 2.

Referring to FIG. 5, an opening for passing the piston 16 is formed in the first locking nut 18. The opening includes a first opening 181 corresponding to the pressure transmission pad 38 and a second opening 182 corresponding to the second piston 40. After the piston 16 is applied with the pressure from the hydraulic ram, the first locking nut 18 is fastened until an upper end of the first opening 181 reaches the pressure transmission pad 38, thereby fixing the position of the piston 16.

Referring back to FIG. 2, the second locking nut 20 is detachably joined to the inside of the second flange 34. For this, a fifth thread 501 may be formed on an inner wall of the second flange 34 and a sixth thread 502 may be formed on an outer wall of the second locking nut 20. The second locking nut 20 serves to fix positions of the support 28 and a fixing member 52 by pressing the fixing member 52 connected to the support 28.

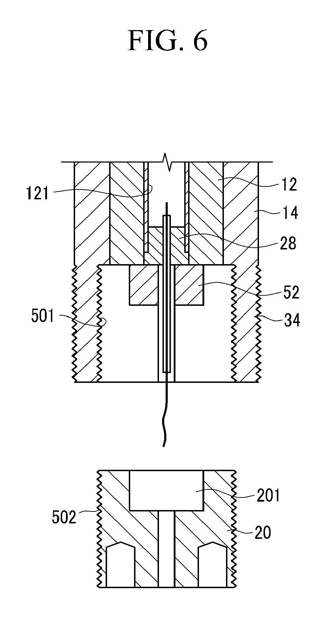

FIG. 6 is a partial cross-sectional view illustrating a state in which the second flange 34 and the second locking nut 20 are separated from each other in the pressure device 100 shown in FIG. 2.

Referring to FIG. 6, the fixing member 52 is fixed to a lower end of the support 28 and has a width larger than the support 28. Accordingly, when the support 28 is pushed into the through-hole 121 of the inner cylinder 12, the fixing member 52 is in close contact with the lower end of the inner cylinder 12. The fixing member 52 may be fabricated by tungsten carbide. A third opening 201 corresponding to the fixing member 52 is formed in the second locking nut 20. The second locking nut 20 is fastened until a lower end of the third opening 201 reaches the fixing member 52, thereby pressurizing the support 28 and the fixing member 52.

Referring back to FIG. 2, the first locking nut 18 and the second locking nut 20 may be fabricated by the same material as the outer cylinder 14 so as to have the same mechanical and thermal properties as the outer cylinder 14. That is, the first and second locking nuts 18 and 20 may also be fabricated by the nickel-chrome-aluminum alloy.

The pressure device 100 includes a lead wire 54 that passes through the support 28, the fixing member 52, and the second locking nut 20, and a protection tube 56 that surrounds the lead wire 54. One end of the lead wire 54 is fixed to the sample 22 in the sealing cap 26 and the other end of the lead wire 54 is connected with a measurement device (not shown) to thereby electrically connect the measurement device with the sample 22.

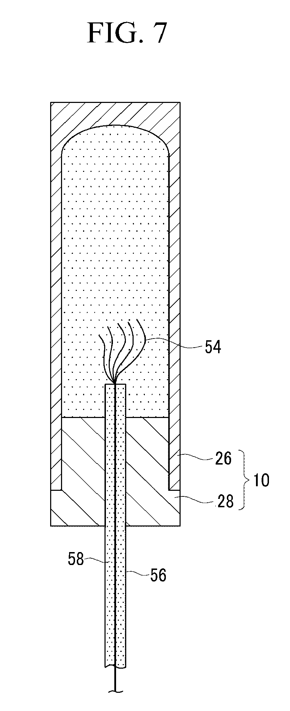

FIG. 7 is a partial cross-sectional view illustrating the sealing member 10, the lead wire 54, and the protection tube 56 in the pressure device 100 shown in FIG. 2.

Referring to FIG. 7, the protection tube 56 passes through the support 28 in close contact with the support 28. The lead wire 54 is positioned inside of the protection tube 56 with being surrounded by an insulating adhesive 58. The lead wire 54 may be a copper wire and may be mounted with being twisted in order to reduce an electromagnetic pickup.

The protection tube 56 may be fabricated by the polymer resin or a metal such as cupro-nickel or stainless steel. The protection tube 56 prevents a short-circuit between the lead wire 54 and the support 28 at a high pressure of 4.0 GPa and a low temperature of 4K and can effectively secure stability of the lead wire 54. However, when the protection tube 56 is fabricated by the metal, the protection tube 56 has conductivity, such that the lead wire 54 and the protection tube 56 must not be in contact with each other.

The insulating adhesive 58 may be an epoxy-based adhesive. After the insulating adhesive 58 is inserted into the protection tube 56 altogether with the lead wire 54, the insulating adhesive 58 is slowly cured and fixed to the protection tube 56. At this time, when bubbles rise in the insulating adhesive 58, the bubbles becomes increasingly larger at the time of applying a high pressure to the pressure device 100 or lowering a temperature, such that the insulating adhesive 58 may be broken. Accordingly, the insulating adhesive 58 may be degassed in a desiccator (not shown) for approximately 60 minutes.

Referring back to FIG. 2, a hollow having a diameter larger than a diameter of the protection tube 56 is formed in the fixing member 52 and the second locking nut 20 and surrounds the protection tube 56. In addition, a cylinder holder 60 supporting the pressure device 100 may be joined to an outer wall of the second flange 34.

The pressure device 100 having the above-mentioned configuration pressurizes the sealing cap 26 with the piston 16 to induce a change of the volume and a change of an electrical state of the sample 22 inside of the sealing cap 26. Therefore, the measurement device (not shown) may measure the change of the electrical state of the sample 22 through the lead wire 54 fixed to the sample 22 and an experimenter can investigate the electrical and magnetic properties through a measurement result.

The pressure device 100 according to the exemplary embodiment has a dual structure of the inner and outer cylinders 12 and 14, such that it is possible to improve the durability by securing high mechanical rigidity. In addition, the inner cylinder 12 is separated from the outer cylinder 14 in accordance with the experimental conditions, such that the inner cylinder 12 can be easily replaced with an inner cylinder 12 of a different material. Further, the sealing member 10 is separated from the inner cylinder 12 and the sealing cap 26 is separated from the support 28 to thereby easily replace the sample 22.

Further, in the pressure device 100 according to the exemplary embodiment, the inner and outer cylinders 12 and 14 and the first and second locking nuts 16 and 18 are fabricated by the nickel-chrome-aluminum alloy which is the high-rigidity and non-magnetic metal, such that the pressure device 100 can effectively endure repetitive thermal cycling and perform accurate measurement under a cryogenic high-pressure and high-magnetic field environment.

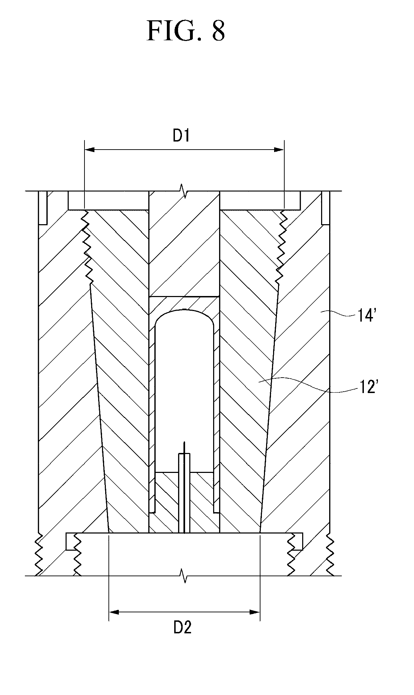

Meanwhile, in FIG. 2, although the outer surface of the inner cylinder 12 and the inner surface of the outer cylinder 14 are formed to be vertical, an outer surface of an inner cylinder 12' and an inner surface of an outer cylinder 14' may be formed to be slant as shown in FIG. 8.

Referring to FIG. 8, the outer surface of the inner cylinder 12' has a diameter that gradually decreases from a pressure applied side to a side opposite to the pressure applied side. In FIG. 8, the pressure applied side is an upper side and the opposite side is a lower side. Accordingly, in the inner cylinder 12', a diameter (D1 of FIG. 8) of the pressure applied side is larger than a diameter (D2 of FIG. 8) of the opposite side. The inner surface of the outer cylinder 14' has the same shape as the outer surface of the inner cylinder 12'.

By the structure shown in FIG. 8, when the pressure is applied by the piston 16, a wedge effect is induced, such that it is possible to effectively prevent the inner cylinder 12' from being deviated outside of the outer cylinder 14'.

While this invention has been described in connection with what is presently considered to be practical exemplary embodiments, it is to be understood that the invention is not limited to the disclosed embodiments, but, on the contrary, is intended to cover various modifications and equivalent arrangements included within the spirit and scope of the appended claims.

While this invention has been described in connection with what is presently considered to be practical exemplary embodiments, it is to be understood that the invention is not limited to the disclosed embodiments, but, on the contrary, is intended to cover various modifications and equivalent arrangements included within the spirit and scope of the appended claims.

## Claims

1. A pressure device for measuring a physical property, comprising:
a sealing member adapted to incorporate a sample and a pressure transmission medium that surrounds the sample;
an inner cylinder that is provided with a through-hole in which the sealing member is installed;
an outer cylinder that surrounds the inner cylinder in close contact with an outer surface of the inner cylinder; and
a piston joined to the through-hole of the inner cylinder to pressurize the sealing member,
wherein the inner cylinder and the outer cylinder are fabricated by a non-magnetic metal, and
the volume and a lattice structure of the sample are changed by pressurizing the sample with the piston to thereby induce a change of electrical and magnetic properties of the sample.

2. The pressue device of claim 1, wherein:
at least one of the inner cylinder and the outer cylinder is fabricated by the nickel-chrome-aluminum alloy.

3. The pressure device of claim 1, wherein:
the outer cylinder is fabricated by a nickel-chrome-aluminum alloy and the inner cylinder is fabricated by any one of a copper-beryllium alloy, maraging steel, and a nickel-cobalt-chrome-molybdenum alloy.

4. The pressure device of claim 1, wherein:
the sealing member includes,
a sealing cap that is fabricated by polytetraflouoroethylene (PTFE), is in closed contact with the piston and of which an end opposite to the piston is opened; and
a support that is fabricated by the copper-beryllium alloy, and is joined to the end of the sealing cap.

5. The pressure device of claim 4, wherein:
an outer diameter of the sealing cap is larger than a diameter of the through-hole, such that the sealing cap presses on the through-hole.

6. The pressure device of claim 4, wherein:
a lubricant containing polytetrafluoroethylene (PTFE) powder is applied between the sealing cap and the inner cylinder.

7. The pressure device of claim 1, wherein:
the inner cylinder is detachably joined to the outer cylinder,
wherein a thread is formed on an outer surface of the inner cylinder and an inner surface of the outer cylinder, such that the inner cylinder and the outer cylinder are joined to each other by screw engagement.

8. The pressure device of claim 1, wherein:
the outer cylinder has a first flange formed at a pressure applied end thereof and a second flange formed at the other end opposite to the first flange, and
the pressure device for measuring a physical property further includes,
a first locking nut that screw-engages with the inside of the first flange to fix a position of the piston; and
a second locking nut that screw-engages with the inside of the second flange to fix a position of the sealing member.

9. The pressure device of claim 8, wherein:
the piston includes,
a first piston joined to the inner cylinder;
a pressure transmission pad connected to the first piston in a pressure applied direction; and
a second piston connected to the pressure transmission pad in the pressure applied direction,
wherein the pressure transmission pad has a width larger than the first and second pistons,
wherein at least one of the first piston, the pressure transmission pad, and the second piston are fabricated by tungsten carbide.

10. The pressure device of claim 9, further comprising:
a piston holder that surrounds a pressure applied end of the second piston,
wherein the piston holder includes,
a back-up member fixed to the end of the second piston; and
a holder body that surrounds the entirety of the back-up member and a portion of the second piston.

11. The pressure device of claim 10, wherein:
the back-up member has a width larger than the second piston and at least one of the second piston and the back-up member is fabricated by tungsten carbide.

12. The pressure device of claim 10, wherein:
the holder body has a width larger than the back-up member, at least one of the second piston and the back-up member is fabricated by tungsten carbide, and the holder body is fabricated by stainless steel.

13. The pressure device of claim 9, wherein:
the first locking nut has a first opening having a shape corresponding to a shape of the pressure transmission pad and a second opening having a shape corresponding to a shape of the second piston.

14. The pressure device of claim 8, further comprising:
a fixing member that is fixed to the sealing member and is in closed contact with the end of the inner cylinder in the inside of the second flange,
wherein the second locking nut has an opening having a shape corresponding to the fixing member.

15. The pressure device of claim 8, wherein:
at least one of the outer cylinder, the first locking nut, and the second locking nut is fabricated by the nickel-chrome-aluminum alloy.

16. The pressure device of claim 14, further comprising:
a lead wire that passes through the sealing member, the fixing member, and the second locking nut, and is electrically connected to the sample; and
a protection tube that surrounds the lead wire with an insulating adhesive interposed therebetween,
wherein the lead wire is a copper wire and is mounted on the protection tube with being twisted, and the insulating adhesive is an epoxy-based adhesive and the protection tube is fabricated by any one of a polymer resin, cupro-nickel, and stainless steel.
